(19)

**Europäisches Patentamt**
**European Patent Office**
**Office européen des brevets**

(11)  **EP 3 636 145 B1**

(12)  **EUROPEAN PATENT SPECIFICATION**

(45) Date of publication and mention
of the grant of the patent:
**31.12.2025 Bulletin 2026/01**

(21) Application number: **19181192.6**

(22) Date of filing: **19.06.2019**

(51) International Patent Classification (IPC):
*A61B 5/021* $^{(2006.01)}$      *A61B 5/024* $^{(2006.01)}$
*A61B 5/00* $^{(2006.01)}$

(52) Cooperative Patent Classification (CPC):
**A61B 5/02116; A61B 5/02416; A61B 5/7235;**
**A61B 5/7242;** A61B 5/681; A61B 5/6898

(54) **APPARATUS AND METHOD FOR ESTIMATING BLOOD PRESSURE**

VORRICHTUNG UND VERFAHREN ZUR SCHÄTZUNG DES BLUTDRUCKS

APPAREIL ET PROCÉDÉ POUR ESTIMER LA PRESSION SANGUINE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **10.10.2018 KR 20180120640**

(43) Date of publication of application:
**15.04.2020 Bulletin 2020/16**

(73) Proprietor: **Samsung Electronics Co., Ltd.**
**Gyeonggi-do 16677 (KR)**

(72) Inventors:
• **PARK, Chang Soon**
**Chungcheongbuk-do (KR)**

• **KWON, Ui Kun**
**Gyeonggi-do (KR)**
• **YOON, Seung Keun**
**Seoul (KR)**
• **JANG, Dae Geun**
**Gyeonggi-do (KR)**

(74) Representative: **Grünecker Patent- und**
**Rechtsanwälte**
**PartG mbB**
**Leopoldstraße 4**
**80802 München (DE)**

(56) References cited:
WO-A1-2018/072175      CN-A- 107 233 087
US-A1- 2005 119 578      US-A1- 2005 261 593
US-A1- 2011 288 422

EP 3 636 145 B1

**Description**

Apparatuses and methods consistent with example embodiments relate to cufflessly estimating blood pressure.

Recently, with the aging population, soaring medical costs, and a lack of medical personnel for specialized medical services, research is being actively conducted on tech convergence for medical devices. Particularly, monitoring of patients' health condition is not limited to places such as hospitals, but is expanding to mobile healthcare fields that may allow the patients to monitor their health condition anywhere and anytime in daily life including their home or office. Typical examples of bio-signals, which indicate the health condition of individuals, include an electrocardiography (ECG) signal, a photoplethysmogram (PPG) signal, an electromyography (EMG) signal, and the like, and various bio-signal sensors are being developed to measure these signals in daily life. Particularly, a PPG sensor may estimate blood pressure of a human body by analyzing a pulse waveform which reflects a condition of the cardiovascular system of the human body.

According to studies on the PPG signal, the entire PPG signal is a superposition of propagation waves starting from the heart toward the distal end portions of the body and reflection waves returning back from the distal end portions. Further, it has been known that information for estimating blood pressure may be obtained by extracting various features related to the propagation waves or the reflection waves.

US 2011/288422 A1 relates to a device and method for estimating central systolic blood pressure based on oscillometric signals from brachial artery by the use of a pressure cuff. The method includes (1) detecting the oscillometric waveform in the cuff; (2) adjusting the oscillometric waveform by the average blood pressure and diastolic blood pressure measured by the cuff to calculate (I) systolic blood pressure of pulse volume recording, (II) last phase systolic blood pressure of pulse volume recording, (III) the value of the area below the waveform during systole and the area below the waveform during diastole dividing the area below the waveform during diastole, and (IV) the pressure of reflected wave hiding beneath the waveform of pressure; and (3) bringing factors (I) to (IV) to a formula, to measure the central arterial blood pressure.

US 2005/119578 A1 relates to an electronic hemomanometer and a blood pressure measuring method. The method includes (1) pressurizing the cuff; (2) detecting a pulse wave generated at the site to be measured occluded by the cuff; (3) detecting a signal which is in turn transmitted to a waveform parameter calculator calculating a waveform parameter representing an amount of feature in waveform of a pulse wave; (4) obtaining an estimated mean arterial pressure; (5) calculating a diastolic blood pressure by conventional oscillometry; (6) calculating an average value of pulse waveform detected by cuff pressure pulse wave detector; (7) correcting waveform distortion; (8) calculating systolic blood pressure using the mean arterial pressure EMAP, the diastolic pressure DBP, the waveform's average value $A_{AV}$ and the correction factor; (9) calculating blood pressure variation range and (10) displaying the result.

CN 107 233 087 A relates to a non-invasive blood pressure measuring device based on the characteristics of photoelectric capacitive pulse wave.

US 2005/261593 A1 relates to a method for measuring an arterial blood pressure of a subject comprising detecting a pulse-wave-related signal of the subject; extracting a feature from the signals; determining a factor that can affect the arterial blood pressure of the subject; and determining the arterial blood pressure based on the feature with an automatic compensation for the factors.

It is therefore the object of the present invention to provide an improved blood pressure estimating apparatus, as well as a corresponding method and non-transitory computer readable storage medium.

This object is solved by the subject matter of the independent claims.

Preferred embodiments are defined by the dependent claims.

According to an aspect of an example embodiment, there is provided a blood pressure estimating apparatus including: a sensor configured to measure a bio-signal waveform; and a processor configured to obtain a first feature based on a first area under the bio-signal waveform in a first time interval, obtain a second feature based on a second area under the bio-signal waveform in a second time which is different from the first interval, and estimate blood pressure based on the first feature and the second feature.

The processor may estimate systolic blood pressure (SBP) and diastolic blood pressure (DBP) by applying a blood pressure estimation model to each of the first feature and the second feature.

The processor may determine a start time and an end time of each of the first time interval and the second time interval based on a bio-signal period of the bio-signal waveform, and a reference starting value and a reference ending value of each of the first time interval and the second time interval.

In this case, the reference starting value and the reference ending value of each of the first time interval and the second time interval may include at least one of a constant value and a variable value which is adaptively changed according to a shape of the bio-signal waveform.

The processor may obtain the first area of the first time interval and the second area of the second time interval by integrating differences between an amplitude value of the bio-signal waveform and an amplitude value of a baseline of the bio-signal waveform, for the first time interval and the second time interval, respectively.

In this case, the amplitude value of the baseline may include at least one of an amplitude value of the bio-signal waveform at a measurement start time of the bio-signal waveform, a minimum amplitude value of the bio-signal waveform, and an amplitude value of a straight line formed by connecting the start time and the end time of each of the first time interval and the second time interval.

The processor may determine a start time and an end time of each of the first time interval and the second time interval based on at least one of a start time and an end time of measurement of the bio-signal waveform, a first time of a position of a pulse waveform component present in the bio-signal waveform, a second time of a dicrotic notch position of the bio-signal waveform, times calculated by multiplying the first and the second times by a predetermined coefficient, and times of an internally dividing point obtained by applying a weighted value to the first and the second times.

The processor may perform differentiation on the bio-signal waveform to obtain a differential signal, and may determine a local minimum point of the differential signal to be the position of the pulse waveform component.

The weighted value may be determined based on at least one of an amplitude of the differential signal and an amplitude of the bio-signal waveform corresponding to the first and the second times.

The processor may obtain the first feature and the second feature by normalizing the first area and the second area based on a first reference value and a second reference value, respectively.

The processor may normalize the first area and the second area by dividing the first area and the second area by the first reference value and the second reference value respectively, or by dividing the first reference value and the second reference value by the first area and the second area, respectively.

The processor may set the first reference value and the second reference value based on at least one of a predetermined value, an amplitude value of the bio-signal waveform in a systolic phase or a diastolic phase of the bio-signal waveform, an amplitude value of an internally dividing point between two pulse waveform components of the bio-signal waveform, and a maximum amplitude value of the bio-signal waveform in the systolic phase or the diastolic phase.

The processor may further obtain a third feature from the bio-signal waveform, and may estimate systolic blood pressure based on the first feature and the third feature and diastolic blood pressure based on the second feature and the third feature.

The processor may obtain the third feature from the bio-signal based on at least one of a shape of the bio-signal waveform, a time value and an amplitude value of a maximum point of the bio-signal waveform, a time value and an amplitude value of a minimum point of the bio-signal waveform, a time value and an amplitude value of a position of a pulse waveform component present in the bio-signal waveform, and an area under the bio-signal waveform in the third interval.

The sensor may include: a light source configured to emit light onto an object; and a detector configured to detect light scattered from the object.

In addition, the blood pressure estimating apparatus may further include an output interface configured to output a processing result of the processor.

According to an aspect of another example embodiment, there is provided a blood pressure estimating method including: measuring a bio-signal waveform; obtaining a first area under the bio-signal waveform in a first time interval and a second area under the bio-signal waveform in a second time interval which is different from the first time interval; obtaining a first feature and a second feature based on the first area and the second area, respectively; and estimating blood pressure based on the first feature and the second feature.

The estimating of the blood pressure may include independently estimating systolic blood pressure (SBP) and diastolic blood pressure (DBP) by applying a blood pressure estimation model to each of the first feature and the second feature.

The obtaining of the first and the second areas may include determining a start time and an end time of each of the first time interval and the second time interval based on a bio-signal period of the bio-signal waveform, and a reference starting value and a reference ending value of each of the first time interval and the second time interval.

The reference starting value and the reference ending value of each of the first time interval and the second time interval may include at least one of a constant value and a variable value which is adaptively changed according to a shape of the bio-signal waveform.

The obtaining of the first and the second areas may include obtaining the first area and the second area by integrating differences between an amplitude value of the bio-signal waveform and an amplitude value of a baseline of the bio-signal waveform, for the first time interval and the second time interval, respectively.

The amplitude value of the baseline may include at least one of an amplitude value of the bio-signal waveform at a measurement start time of the bio-signal waveform, a minimum amplitude value of the bio-signal waveform, and an amplitude value of a straight line formed by connecting the start time and the end time of each of the first time interval and the second time interval.

The obtaining of the first and the second areas may include determining a start time and an end time of each of the first time interval and the second time interval based on at least one of a start time and an end time of measurement of the bio-signal waveform, a first time of a position of a pulse waveform component present in the bio-signal waveform, a second time of a dicrotic notch position of the bio-signal waveform, times calculated by multiplying the first and the second times by a predetermined coefficient, and times of an internally dividing point obtained by applying a weighted value to the first and the second times.

The obtaining of the first feature and the second feature may include normalizing the first area and the second area based on a first reference value and a second reference value, respectively.

The obtaining of the first feature and the second feature may include setting the first reference value and the second reference value based on at least one of a predetermined value, an amplitude value of the bio-signal waveform in a systolic phase or a diastolic phase of the bio-signal waveform, an amplitude value of an internally dividing point between two pulse waveform components of the bio-signal waveform, and a maximum amplitude value of the bio-signal waveform in the systolic phase or the diastolic phase.

In addition, the blood pressure estimating method may further include obtaining a third feature from the bio-signal waveform, wherein the estimating of the blood pressure may include estimating systolic blood pressure based on the first feature and the third feature and diastolic blood pressure based on the second feature and the third feature.

The obtaining of the third feature may include obtaining the third feature from the bio-signal waveform based on at least on one or two or more of a shape of the bio-signal waveform, a time value and an amplitude value of a maximum point of the bio-signal waveform, a time value and an amplitude value of a minimum point of the bio-signal waveform, a time value and an amplitude value of a position of a pulse waveform component present in the bio-signal waveform, and an area under the bio-signal waveform in a third time interval.

Moreover, the blood pressure estimating method may further include outputting an estimation result of blood pressure.

According to an aspect of another example embodiment, there is provided a non-transitory computer readable storage medium storing a program that is executable by a computer to perform a method including: obtaining a bio-signal waveform; setting a systolic time interval and a diastolic time interval of the bio-signal waveform to be different from each other; calculating a first area under the bio-signal waveform in the systolic time interval, and a second area under the bio-signal waveform in the diastolic time interval; and estimating a blood pressure based on the first area in the systolic time interval and the second area in the diastolic time interval.

BRIEF DESCRIPTION OF THE DRAWINGS

The above and/or other aspects will be more apparent by describing certain example embodiments, with reference to the accompanying drawings, in which:

FIGS. 1A and 1B are block diagrams illustrating a blood pressure estimating apparatus according to example embodiments;

FIGS. 2A and 2B are block diagrams illustrating a processor of FIGS. 1A and 1B;

FIGS. 3A, 3B, 3C, 3D, and 3E are diagrams explaining extracting features for estimating systolic blood pressure (SBP) and diastolic blood pressure (DBP) according to example embodiments;

FIG. 4 is a flowchart illustrating a blood pressure estimating method according to an example embodiment;

FIG. 5 is a flowchart illustrating a blood pressure estimating method according to another example embodiment;

FIGS. 6A and 6B are diagrams illustrating a wearable device according to an example embodiment; and

FIG. 7 is a diagram illustrating a smart device for estimating blood pressure according to an example embodiment.

DETAILED DESCRIPTION

Example embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'part' or 'module', etc., should be understood as a unit that performs at least one function or operation and that may be embodied as hardware, software, or a combination thereof.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

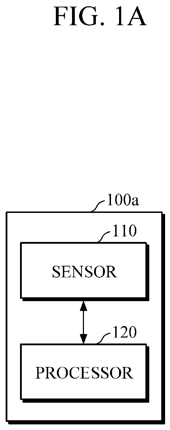

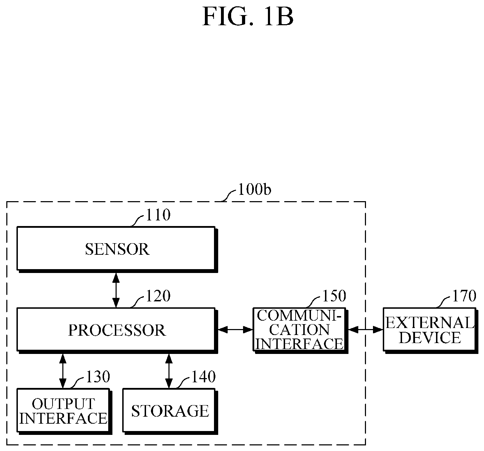

FIGS. 1A and 1B are block diagrams illustrating a blood pressure estimating apparatus according to example embodiments. The blood pressure estimating apparatuses 100a and 100b may be embedded in an electronic device, such as a smartphone, a tablet PC, a desktop computer, a laptop computer, and the like, or in a wearable device that may be worn on an object. In particular, examples of the wearable device may include a wristwatch-type wearable device, a bracelet-type wearable device, a wristband-type wearable device, a ring-type wearable device, a glasses-type wearable device, a hairband-type wearable device, or the like, but the wearable device is not limited thereto, and may be embedded in a medical device manufactured for use in medical institutions to measure and analyze bio-information.

Referring to FIGS. 1A and 1B, each of the blood pressure estimating apparatuses 100a and 100b includes a sensor 110 and a processor 120.

The sensor 110 may measure a bio-signal from an object. In particular, the bio-signal may be a pulse wave signal including a photoplethysmogram (PPG) signal. However, the bio-signal is not limited thereto, and may include various bio-signals, such as an electrocardiography (ECG) signal, an electromyography (EMG) signal, and the like, which may be modeled by adding a plurality of waveform components. The object may be a body part which comes into contact with or is adjacent to the sensor 110, and may be a body part where pulse waves may be easily measured. For example, the object may be an area of skin on the wrist that is adjacent to the radial artery or a human skin area through which veins or capillaries pass. However, the object is not limited thereto, and may be peripheral body portions, such as fingers, toes, and the like, which have a high density of blood vessels.

The sensor 110 may include a light source and a detector. The light source may emit light onto the object, and the detector may detect light scattered or reflected from the object. The light source may include a light emitting diode (LED), a laser diode (LD), a fluorescent body, and the like, and may be arranged in one or two or more arrays. The detector may include one or more pixels, each of which includes a photo diode, a photo transistor (PTr), an image sensor, and the like, which detects light and converts the detected light into an electric signal. The sensor 110 may be implemented as a spectrometer.

The processor 120 may be electrically connected to the sensor 110. The processor 120 may control the sensor 110 in response to a request for estimating blood pressure, and may receive a bio-signal from the sensor 110. The request for estimating blood pressure may be input by a user, or may be generated at predetermined estimation intervals. Upon receiving an electrical bio-signal from the sensor 110, the processor 120 may perform preprocessing such as filtering for removing noise, amplification of the bio-signal, conversion of the bio-signal into a digital signal, and the like.

The processor 120 may estimate blood pressure based on the bio-signal received from the sensor 110. In particular, the processor 120 may independently estimate systolic blood pressure (SBP) and diastolic blood pressure (DBP) by analyzing a waveform of a bio-signal. For example, based on the area under a waveform of a bio-signal, the processor 120 may obtain a first feature for estimating SBP and a second feature for estimating DBP, and may estimate SBP by using the first feature and DBP by using the second feature.

Referring to FIG. 1B, the blood pressure estimating apparatus 100b may further include an output interface 130, a storage 140, and a communication interface 150.

The output interface 130 may output results processed by the sensor 110 and the processor 120. For example, the output interface 130 may visually output an estimated blood pressure value by using a display screen, or may output the estimated blood pressure value in a non-visual manner through voice, vibration, tactile sensation, and the like, by using a speaker, a haptic motor, and the like. The output interface 130 may divide a display area into two or more areas according to a setting, and may output a bio-signal graph used for estimating blood pressure, a blood pressure estimation result, and the like in a first area, and a blood pressure estimation history in the form of a graph and the like in a second area. In this case, if an estimated blood pressure value falls outside a normal range, the output interface 130 may output warning information in various manners, such as highlighting an abnormal value in red and the like, displaying the abnormal value along with a normal range, outputting a voice warning message, adjusting a vibration intensity, and the like.

The storage 140 may store results processed by the sensor 110 and the processor 120. Further, the storage 140 may store various criteria required for estimating blood pressure. For example, the criteria may include user feature information such as a user's age, gender, health condition, and the like. In addition, the criteria may also include information such as a reference blood pressure value, a blood pressure estimation model, a blood pressure estimation period, and the like. Further, the criteria may include a method of setting an interval for calculating an area, a start reference value and an end reference value of each interval, a baseline for calculating an area, a first reference value and a second reference value for normalization, and the like, but are not limited thereto.

In particular, the storage 140 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but the storage medium is not limited thereto.

The communication interface 150 may communicate with an external device 170 using wired or wireless communication techniques under the control of the processor 120, and may transmit and receive various data to and from the external device 170. For example, the communication interface 150 may transmit a blood pressure estimation result to the external device 170, and may receive various criteria required for estimating blood pressure from the external device 170. Examples of the external device 170 may include a cuff-type blood pressure measuring device, and an information processing device such as a smartphone, a tablet PC, a desktop computer, a laptop computer, and the like.

In particular, examples of the communication techniques may include Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G communication, 4G communication, 5G communication, and the like. However, this is merely an example and is not intended to be limiting.

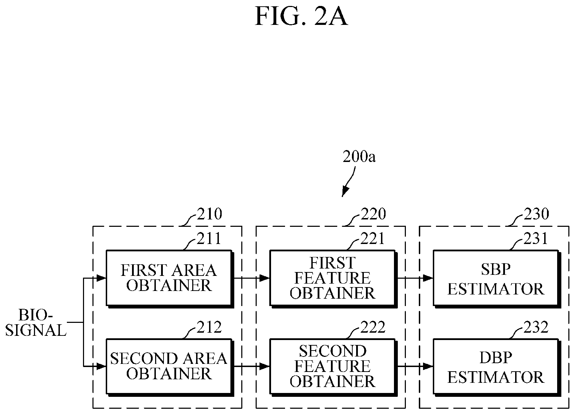

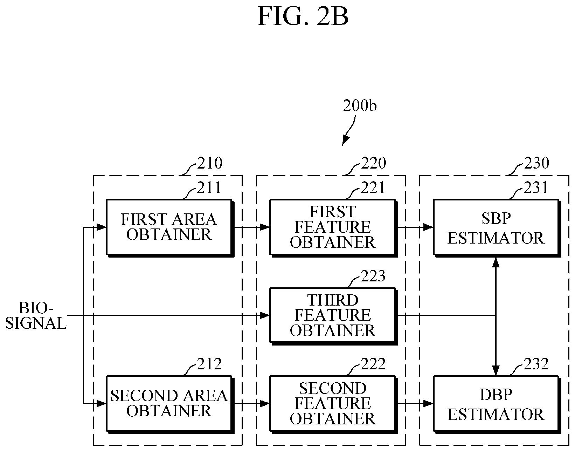

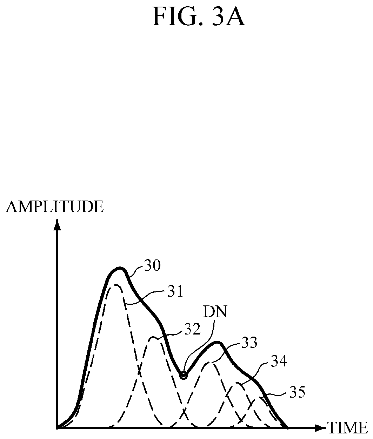

FIGS. 2A and 2B are block diagrams illustrating a processor according to example embodiments of FIGS. 1A and 1B. FIGS. 3A to 3E are diagrams explaining extracting features for estimating systolic blood pressure (SBP) and diastolic blood pressure (DBP) according to example embodiments.

FIG. 3A illustrates a measured pulse wave signal 30 formed by superposition of five pulse waveforms 31, 32, 33, 34, and 35 according to an example embodiment. The pulse wave signal is a superposition of propagation waves of a pumping pulse and reflection waves, wherein the pumping pulse represents blood flow that starts from the heart toward the distal end portions of the body, and the reflection waves represent blood flow that returns back from the distal end portions. Based on the combination of time and amplitude values of the pulse waveforms 31, 32, 33, 34, and 35, features having a high correlation with blood pressure may be extracted. For example, the first, second and third pulse waveforms 31-33 may be used to estimate blood pressure. Pulse waveforms after the third pulse waveform 33 may not be observed depending on individuals in some cases and are difficult to be found due to noise, or have a low correlation with estimation of blood pressure.

Among the pulse waveforms 31, 32, 33, 34, and 35 of the pulse wave signal 30, the first pulse waveform 31 and the second pulse waveform 32 may be present in a systolic phase, and the pulse waveform 34 and 35 following the third pulse waveform 33 may be present in a diastolic phase. Here, the systolic and the diastolic phases may be divided based on a position of a downwardly concave dicrotic notch (DN) which is located between the second pulse waveform 32 and the third pulse waveform 33. For example, the systolic phase may correspond to the bio-signal between a start time $T_{start}$ of

the bio-signal and a time $T_{DN}$ when the DN occurs, and the diastolic phase may correspond to the bio-signal between the time $T_{DN}$ and an end time $T_{end}$ of the bio-signal. In defining the systolic phase and the diastolic phase, an offset time $T_{offset}$ may be added to the start time $T_{start}$ and may be subtracted from the end time $T_{end}$.

Referring to FIG. 2A, a processor 200a includes an area obtainer 210, a feature obtainer 220, and a blood pressure estimator 230.

The area obtainer 210 may include a first area obtainer 211 and a second area obtainer 212. The first area obtainer 211 may determine a first area under a bio-signal waveform in a first interval to estimate SBP. The area under the bio-signal waveform may be referred to Area Under the Curve (AUC). For example, the area obtainer 210 may obtain the first area by using the following Equation 1.

[Equation 1]

$$Asys = \sum_{t=T\_sys\_1}^{t=T\_sys\_2} (P(t) - P_{base})$$

Herein, Asys denotes the first area of the first interval which is to be used to estimate SBP; T_sys_1 and T_sys_2 denote a start time and an end time of the first interval; P(t) denotes an amplitude value at a time (t); and $P_{base}$ denotes a baseline amplitude value. In this case, the amplitude value of the baseline may be an amplitude value at the initial start time of the bio-signal or an amplitude value in the entire interval of the bio-signal; or may be an amplitude value of a straight line formed by connecting the start time and the end time, e.g., an amplitude value corresponding to the time (t) on the straight line with respect to the time (t), but the amplitude value of the baseline is not limited thereto.

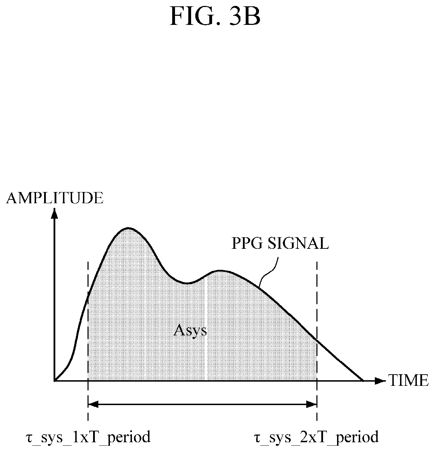

The first area obtainer 211 may analyze the bio-signal to determine the start time and the end time of the first interval. For example, the first area obtainer 211 may obtain the start time of the first interval by multiplying a bio-signal period by a reference starting value, and may obtain the end time of the first interval by multiplying the bio-signal period by a reference ending value, as represented by the following Equation 2. FIG. 3B illustrates an example of obtaining the start time and the end time of the first interval of the PPG signal, and obtaining an area Asys between the start time to the end time, as represented by the following Equation 2

[Equation 2]

$$T_{\_sys\_1} = \tau\_sys\_1 \times T\_period$$
$$T_{\_sys\_2} = \tau\_sys\_2 \times T\_period$$

Herein, $\tau$_sys_1 and $\tau$_sys_2 denote the reference starting value and the reference ending value of the first interval respectively; and T_period denotes the period of the bio-signal.

The reference starting value and the reference ending value may be values predetermined through preprocessing. For example, the reference starting value may be selected from a range of 0 to 0.3 and the reference ending value may be selected from a range of 0.7 to 1.0, so that the first interval of the bio-signal may include most of the entire interval thereof. The values are merely examples for convenience of explanation, and are not limited thereto. In this case, the reference starting value and the reference ending value may be predetermined for each user, or for groups classified according to various criteria such as age, gender, health condition, and the like.

Alternatively, the reference starting value and the reference ending value may be values which are adaptively changed according to a shape of a bio-signal. That is, once a bio-signal is measured, the first area obtainer 211 may analyze the measured bio-signal to adjust the reference starting value and the reference ending value. For example, in the case where a heart rate, extracted from the bio-signal, increases higher or decreases lower than a threshold, or in the case where it is determined that a rate of change in the bio-signal over time is greater than a reference rate, the first area obtainer 211 may increase or decrease the reference starting value or the reference ending value according to a predetermined adjustment rate. In this case, the adjustment rate may be set based on comparison between an increase/decrease in the heart rate and the threshold, and/or comparison between the rate of change in the bio-signal over time and the reference rate.

In another example, the first area obtainer 211 may obtain the start time and the end time of the first interval by using time values associated with the pulse waveform components present in the bio-signal. In particular, the first area obtainer 211 may perform secondary differentiation on the bio-signal, and may detect a local minimum point of the differential signal, to determine the position of the local minimum point as a position of the pulse waveform component.

For example, the time values associated with the pulse waveform components may include a time of a position of the pulse waveform component, a time of a position of the DN, a time calculated by multiplying the times by a predetermined coefficient, a time of an internally dividing point obtained by applying a weighted value to the times, a start time and an end time of a bio-signal period, a time calculated by linearly combining at least some of the times, and the like. In this case, the weighted value to be applied to the times may be determined by using an amplitude of a differential signal of positions corresponding to the times, and/or an amplitude of the bio-signal. For example, the amplitude of the differential signal and/or the amplitude of the bio-signal, corresponding to each of the times, may be set as a weighted value to be applied to each time, so that a higher weighted value is applied to time values corresponding to a higher amplitude of a differential signal and/or a higher amplitude of the bio-signal, and an internally dividing point may be obtained by integrating the time values, to which weighted values are applied, and by dividing the added value by the sum of the weighed values. However, the weighted value is not limited thereto.

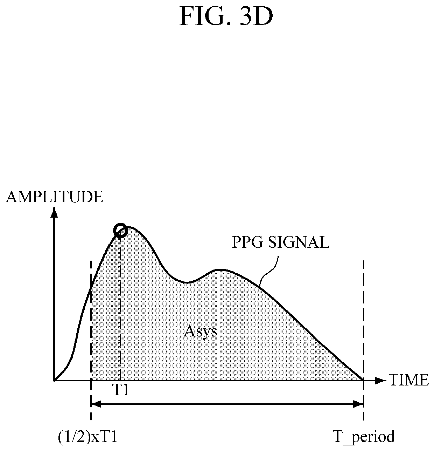

FIG. 3D illustrates an example of determining, as a start time of a first interval, a value $1/2 \times T1$ calculated by multiplying a time T1, corresponding to a position of a first pulse waveform component of a PPG signal, by a predetermined coefficient (1/2); determining, as an end time of the first interval, a time T_period corresponding to an end time of a period of the PPG signal; and obtaining an area Asys of the first interval.

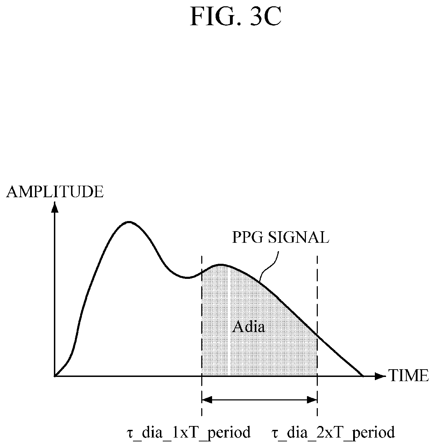

In order to estimate DBP, the second area obtainer 211 may obtain a second area under a bio-signal waveform in a second interval. In this case, the second interval may be an interval which is different from the first interval on a time axis of the bio-signal. For example, the second area obtainer 211 may obtain the second area of the second interval by using the following Equations 3 and 4. FIG. 3C illustrates an example of obtaining a start time and an end time of the second interval of the PPG signal using the following Equation 4, and obtaining an area Adia between the start time and the end time.

[Equation 3]

$$Adia = \sum_{t=T\_dia\_1}^{t=T\_dia\_2} (P(t) - P_{base})$$

[Equation 4]

$$T_{dia\_1} = \tau\_dia\_1 \times T\_period$$
$$T_{dia\_2} = \tau\_dia\_2 \times T\_period$$

Herein, Adia denotes the second area of the second interval for estimating DBP; T_dia_1 and T_dia_2 denote a start time and an end time of the second interval; P(t) denotes an amplitude value at a time (t); and $P_{base}$ denotes an amplitude value of a baseline. In this case, the amplitude value of the baseline may be an amplitude value of the initial start time or a minimum amplitude value in the entire interval of the bio-signal; or may be an amplitude value of a straight line formed by connecting the start time and the end time, e.g., an amplitude value corresponding to the time (t) on the straight line with respect to the time (t), but the amplitude value of the baseline is not limited thereto. Further, $\tau$_dia_1 and $\tau$_dia_2 denote a reference starting value and a reference ending value of the second interval respectively; and T_period denotes the period of the bio-signal.

As in the first area obtainer 211 described above, the second area obtainer 212 may set predetermined fixed values as the reference starting value and the reference ending value. For example, the second area obtainer 212 may set an interval, which is 0.4 to 0.9 times the period of the bio-signal, as the second interval, so that the diastolic interval may be mainly used for estimating DBP. For example, the second area obtainer 212 may set 0.5 as a reference starting value, and 0.8 as a reference ending value. However, the values are merely examples for convenience of understanding, and are not limited thereto. In another example, the second area obtainer 212 may analyze the waveform of the bio-signal to adaptively adjust the reference starting value and the reference ending value, which is described above with reference to the first area obtainer 211, such that detailed description thereof will be omitted.

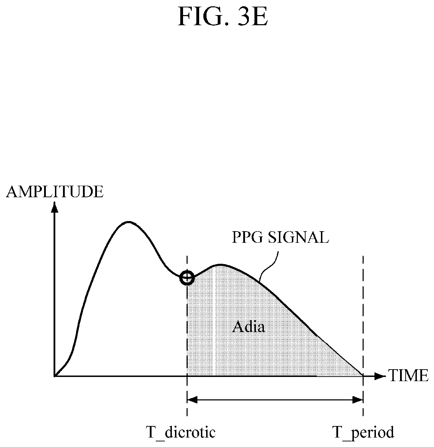

Further, instead of obtaining the start time and the end time by using the above Equation 4, the second area obtainer 212 may obtain the start time and/or the end time of the second interval by using time values associated with the pulse waveform components present in the bio-signal, which is described above with reference to the first area obtainer 211, such that detailed description thereof will be omitted. FIG. 3E illustrates an example of determining a time T_dicrotic, corresponding to the position of the DN of the PPG signal, as the start time of the second interval, and a time T_period corresponding to the end of the period of the bio-signal as the end time of the second interval, and obtaining an area Adia of the second interval.

The feature obtainer 220 may extract features for estimating blood pressure by using the area obtained by the area obtainer 210. The feature obtainer 220 may include a first feature obtainer 221 and a second feature obtainer 222 as illustrated therein.

The first feature obtainer 221 may obtain a first feature for estimating SBP based on the area of the first interval obtained by the first area obtainer 211. For example, the first feature obtainer 221 may obtain the first area of the first interval as it is as the first feature for estimating SBP. In another example, the first feature obtainer 221 may normalize the area of the first interval by using a predetermined first reference value, and may obtain the normalized value as the first feature. For example, the first feature obtainer 221 may normalize the first area of the first interval by dividing the first area of the first interval by the first reference value, or conversely, by dividing the first reference value by the first area of the first interval. In this case, the first reference value may include a bio-signal amplitude value of the position of the first pulse waveform component in the systolic phase, a bio-signal amplitude value of the position of the second pulse waveform component in the systolic phase, a bio-signal amplitude value of an internally dividing point between the position of the first pulse waveform component and the position of the second pulse waveform component, a maximum amplitude value in the systolic phase, and the like. However, the first reference value is not limited thereto.

The second feature obtainer 222 may obtain a second feature for estimating DBP based on an second area of the second interval obtained by the second area obtainer 212. As in the first feature obtainer 221, the second feature obtainer 222 may obtain the second area of the second interval as it is as the second feature, or may obtain the second feature by normalizing the second area of the second interval by using a second reference value. In this case, the second reference value may be the same as or different from the first reference value, and may include a pulse waveform component in the diastolic interval, i.e., an amplitude value of the position of the third pulse waveform component, an amplitude value of the position of the fourth pulse waveform component, an amplitude value of an internally dividing point between the position of the third pulse waveform component and the position of the fourth pulse waveform component, a maximum amplitude value in the diastolic interval, and the like. However, the second reference value is not limited thereto.

The blood pressure estimator 230 may estimate blood pressure by using the first feature and the second feature obtained by the feature obtainer 220. The blood pressure estimator 230 may independently estimate SBP and DBP. To this end, the blood pressure estimator 230 may include an SBP estimator 231 and a DBP estimator 232.

The SBP estimator 231 may estimate SBP based on the first feature. For example, the SBP estimator 231 may estimate SBP by applying an SBP estimation model as represented by the following Equation 5

[Equation 5]

$$SBP = a_{sys} \times f_1 + b_{sys}$$

Herein, the SBP denotes systolic blood pressure; $a_{sys}$ and $b_{sys}$ denote predetermined coefficients for estimating SBP; and $f_1$ denotes the first feature.

The DBP estimator 232 may estimate DBP based on the second feature. For example, the DBP estimator 232 may estimate DBP by applying a DBP estimation model as represented by the following Equation 6.

[Equation 6]

$$DBP = a_{dia} \times f_2 + b_{dia}$$

Herein, the DBP denotes diastolic blood pressure; $a_{dia}$ and $b_{dia}$ denote predetermined coefficients for estimating DBP; and $f_2$ denotes the second feature. In this case, the coefficients for estimating SBP may be the same as or different from the coefficients for estimating DBP.

Referring to FIG. 2B, a processor 200b includes an area obtainer 210, a feature obtainer 220, and a blood pressure estimator 230. In the embodiment, the feature obtainer 220 may further include a third feature obtainer 223 as illustrated in FIG. 2B. Detailed description of components described above with reference to FIG. 2A will be omitted.

The third feature obtainer 223 may obtain an additional third feature, which may be applied collectively for estimating both SBP and DBP, from a bio-signal. For example, the third feature obtainer 223 may obtain a third feature from the bio-signal by combining one or two or more of the following: a shape of a bio-signal waveform, a time value and an amplitude value of a maximum point, a time value and an amplitude value of a minimum point, a time value and an amplitude value of a position of the pulse waveform component present in the bio-signal, and an third area of a third interval of the bio-signal. However, the third feature is not limited thereto, and the third feature obtainer 223 may obtain the third feature by combining the first feature and the second feature by, for example, multiplying the first feature by the second feature. In this case, the first feature and the second feature may be combined in various manners, such as addition, subtraction, division, multiplication, logarithmic value, regression equation, and a combination thereof, with no specific limitation.

The following Equation 7 represents an example of obtaining the third feature by combining bio-signal amplitude values P1, P2, and P3 corresponding to positions of the first, the second, and the third pulse waveform components.

[Equation 7]

$$f_3 = \frac{P1 + P2}{P3}$$

The SBP estimator 231 may combine the first feature, which is obtained by the first feature obtainer 221, and the third feature which is obtained by the third feature obtainer 223, and may estimate SBP by applying a blood pressure estimation model represented by the following Equation 8.

[Equation 8]

$$SBP = a_{sys} \times (w_1 f_1 + w_3 f_3) + b_{sys}$$

Herein, the SBP denotes systolic blood pressure; $a_{sys}$ and $b_{sys}$ denote predetermined coefficients for estimating SBP; $f_1$ and $f_3$ denote the first and the third

features respectively; and $w_1$ and $w_3$ denote weighted values applied to the first and the third features respectively. The weighted values may not be applied as needed, and may be applied to any one of the features.

The DBP estimator 232 may combine the second feature, which is obtained by the second feature obtainer 222, and the third feature which is obtained by the third feature obtainer 223, and may estimate SBP by applying a blood pressure estimation model such as the following Equation 9.

[Equation 9]

$$DBP = a_{dia} \times (w_2 f_2 + w_3 f_3) + b_{dia}$$

Herein, the DBP denotes diastolic blood pressure; $a_{dia}$ and $b_{dia}$ denote predetermined coefficients for estimating DBP; $f_2$ and $f_3$ denote the first and the third features respectively; and $w_2$ and $w_3$ denote weighted values applied to the second and the third features respectively. The weighted values may not be applied as needed, and may be applied to any one of the features.

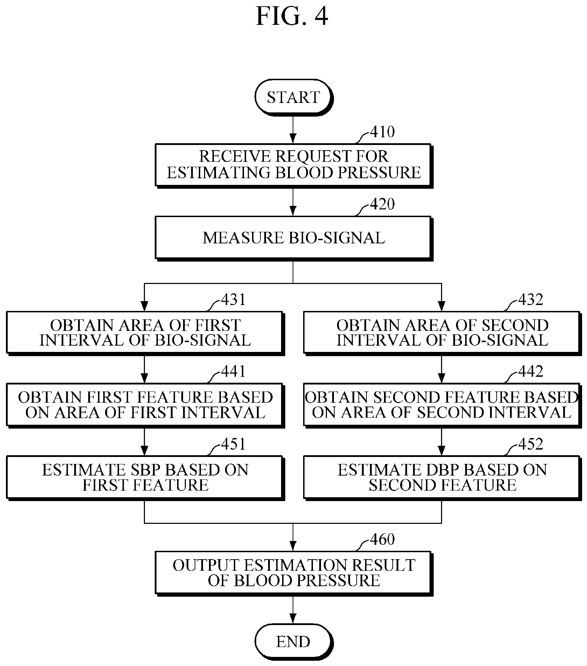

FIG. 4 is a flowchart illustrating a blood pressure estimating method according to an example embodiment. The embodiment of FIG. 4 may be an example of a blood pressure estimating method performed by the blood pressure estimating apparatuses 100a and 100b.

The blood pressure estimating apparatus may receive a request for estimating blood pressure in operation 410. The blood pressure estimating apparatus may provide an interface for interaction with a user. The user may request estimation of blood pressure through the interface provided by the blood pressure estimating apparatus. Alternatively, the blood pressure estimating apparatus may receive the request for estimating blood pressure from an external device. In particular, the request for estimating blood pressure may include a request for providing an estimation result of blood pressure. In the case where the external device includes a blood pressure estimation algorithm, the request for estimating blood pressure may include a request for providing obtained features. Examples of the external device may include a smartphone, a tablet PC, a laptop computer, a wearable device, and the like.

Then, the blood pressure estimating apparatus may measure a bio-signal in operation 420. The blood pressure estimating apparatus may control a sensor to measure a bio-signal, including a pulse wave signal, from an object.

Subsequently, the blood pressure estimating apparatus may analyze the bio-signal to obtain an first area of a first interval and an second area of a second interval of the bio-signal in operations 431 and 432. In this case, the first interval for estimating SBP and the second interval for estimating DBP may be intervals in different ranges on a time axis of a bio-signal. For example, the blood pressure estimating apparatus may obtain the first area and the second area by integrating differences between an amplitude value of each time, ranging from a start time to an end time of each interval, and an amplitude value of a baseline. In this case, the start time and the end time of each interval may be determined by using a reference starting value and a reference ending value, which are predefined for each interval, a bio-signal period, and the like. Further, the reference starting value and the reference ending value may be adaptively adjusted by analyzing the shape of a bio-signal. Alternatively, the start time and the end time of each interval may also be determined by using time values associated with pulse waveform components present in the bio-signal.

Then, the blood pressure estimating apparatus may obtain a first feature based on the first area of the first interval in operation 441, and may obtain the second feature based on the second area of the second interval in operation 442. For example, the blood pressure estimating apparatus may obtain the first feature by normalizing the first area of the first interval using a first reference value, and may obtain the second feature by normalizing the second area of the second interval using a second reference value. In this case, the first reference value and the second reference value may be different from each other, and may be determined by combining amplitude values of the positions of the pulse waveform components, an amplitude value of the bio-signal at the internally dividing point of the positions of the pulse waveform components, and the like.

Subsequently, the blood pressure estimating apparatus may estimate SBP based on the first feature in operation 451, and may estimate DBP based on the second feature in operation 452. As described above, the blood pressure estimating apparatus may estimate blood pressure by inputting each feature into a blood pressure estimation model. In this case, the blood pressure estimation model may be defined differently for SBP and DBP.

Next, the blood pressure estimating apparatus may output an estimation result of blood pressure in operation 460. For example, the blood pressure estimating apparatus may use various visual methods to output the estimation result of blood pressure through a visual output device such as a display and the like. Alternatively, the blood pressure estimating apparatus may output the estimation result of blood pressure in a non-visual manner through voice, vibration, tactile sensation, and the like, by using a speaker and/or a haptic motor. Further, the blood pressure estimating apparatus may determine a user's health condition based on estimated bio-information, and may provide guide information, such as a warning, a response action, and the like, based on the determination.

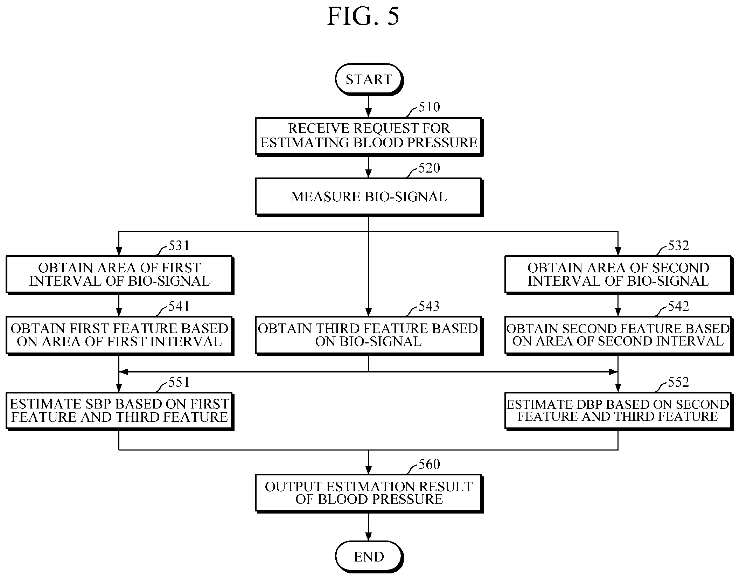

FIG. 5 is a flowchart illustrating a blood pressure estimating method according to another example embodiment. The embodiment of FIG. 5 may be an example of a blood pressure estimating method performed by the blood pressure estimating apparatuses 100a and 100b of FIGS. 1A and 1B.

Upon receiving a request for estimating blood pressure in operation 510, the blood pressure estimating apparatus may measure a bio-signal in operation 520.

Then, the blood pressure estimating apparatus may analyze the bio-signal to obtain an first area of a first interval for estimating SBP and an second area of a second interval for estimating DBP in operations 531 and 532.

Subsequently, the blood pressure estimating apparatus may obtain a first feature based on the first area of the first interval in operation 541 and may obtain a second feature based on the second area of the second interval in operation 542. Further, the blood pressure estimating apparatus may analyze the bio-signal, and may obtain a third feature which may be applied collectively for estimating both SBP and DBP in operation 543. In this case, the blood pressure estimating apparatus may obtain the third feature by combining the first feature and the second feature, and by combining time or amplitude values of positions of pulse waveform components present in the bio-signal.

Next, the blood pressure estimating apparatus may estimate SBP based on the first feature and the third feature in operation 551, and may estimate DBP based on the second feature and the third feature in operation 552. In this case, a predefined blood pressure estimation model may be applied, which may be defined differently for SBP and DBP.

Then, the blood pressure estimating apparatus may output an estimation result of blood pressure to a user by using various visual/non-visual devices and methods in operation 560.

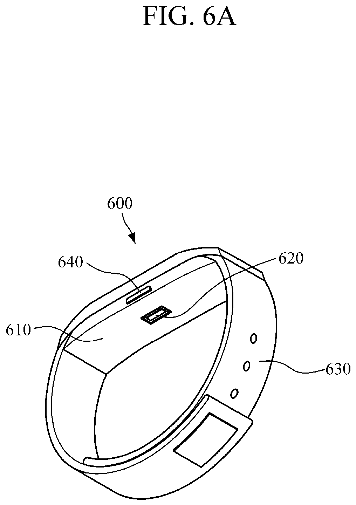

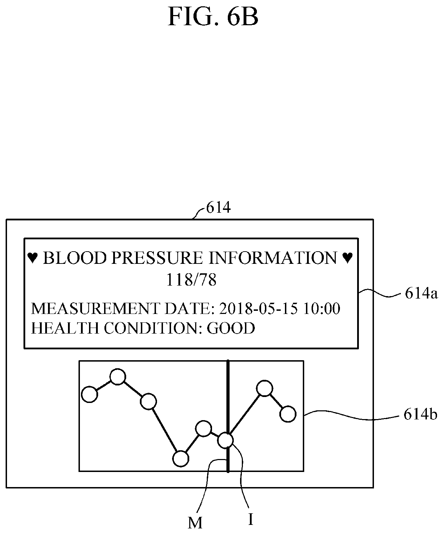

FIGS. 6A and 6B are diagrams illustrating a wearable device according to an example embodiment. Various embodiments of the above-described blood pressure estimating apparatuses 100a and 100b may be embedded in a smart watch worn on the wrist or a smart band-type wearable device. However, this is merely examples for convenience of explanation, and the blood pressure estimating apparatuses 100a and 100b may be applied to an information processing terminal such as a smartphone, a tablet PC, a laptop computer, a desktop computer, and the like.

Referring to FIGS. 6A and 6B, the wearable device 600 includes a main body 610 and a strap 630.

The main body 610 may be formed to have various shapes, and may include modules which are mounted inside or outside of the main body 610 to perform the aforementioned function of estimating blood pressure as well as various other functions. A battery may be embedded in the main body 610 or the strap 630 to supply power to various modules of the wearable device 600.

The strap 630 may be connected to the main body 610. The strap 630 may be flexible, so as to be bent around a user's wrist. The strap 630 may be bent in a manner that allows the strap 630 to be detached from the user's wrist or may be formed as a band that is not detachable. Air may be injected into the strap 630 or an airbag may be included in the strap 630, so that the strap 630 may have elasticity according to a change in pressure applied to the wrist, and the change in pressure of the wrist may be transmitted to the main body 610.

The main body 610 may include a sensor 620 for measuring a bio-signal. The sensor 620 may be mounted on a rear surface of the main body 610, which comes into contact with a user's wrist, and may include a light source for emitting light onto the skin of the wrist and a detector for detecting light scattered or reflected from the object. The sensor 620 may further include a contact pressure sensor for measuring contact pressure of the object.

A processor may be mounted in the main body 610. The processor may be electrically connected to various modules, mounted in the wearable deice 600, to control operations thereof. In addition, the processor may estimate blood pressure by using the bio-signal measured by the sensor 620. As described above, the processor may obtain an area for estimating SBP and an area for estimating DBP from the bio-signal, and may independently estimate SBP and DBP based on each of the areas.

In the case where the sensor 620 includes a contact pressure sensor, the processor may monitor a contact state of the object based on contact pressure between the wrist and the sensor 620, and may provide a user with a guide to a contact position and/or a contact state through a display.

Further, the main body 610 may include a storage which stores a processing result of the processor and various types of information. In this case, various types of information may include criteria for estimating blood pressure as well as information associated with functions of the wearable device 600.

In addition, the main body 610 may also include a manipulator 640 which receives a control command of a user and transmits the received control command to the processor. The manipulator 640 may include a power button to input a command to turn on/off the wearable device 600.

With reference to FIGS. 6A and 6B, a display 614 may be mounted on a front surface of the main body 610, and may include a touch panel for touch input. The display 614 may receive a touch input from a user, may transmit the received touch input to the processor, and may display a processing result of the processor.

For example, the display 614 may display the estimated blood pressure information. In this case, the display 614 may also display additional information such as a blood pressure estimation date, a health condition, and the like. When a user requests detailed information by operating the manipulator 640 or by touching the display 614 for touch input, the display 614 may output detailed information in various manners.

Referring to FIG. 6B, the display 614 may output detailed information in a first region 614a of the display 614 and may output a blood pressure history graph in a second region 614b of the display 614. In this case, the blood pressure history graph may include an object (e.g., a figure such as a circle, a square, etc.) which indicates a blood pressure estimation time. Further, an identification mark M, indicating an object I currently selected by a user, may be displayed on the blood pressure history graph. The identification mark M is shown as a vertical line, but is not limited thereto, and may have various shapes, such as a polygonal shape including a circular shape, a square shape, and the like, an arrow indicating a position, and the like. Once the blood pressure history graph is displayed in the second region 614b, detailed information may be output in the first region 614a when a user touches the object I of a specific time, or moves the graph to the right and left sides to place the object I of the specific time on the identification mark M. In this case, information, such as an estimated blood pressure value, a measurement date, a health condition at the time point, and the like, may be output in the first region 614a. However, the information output in the first region 614a is not limited thereto.

Moreover, a communication interface, provided for communication with an external device, such as a mobile terminal of a user, may be mounted in the main body 610. The communication interface may transmit an estimation result of bio-information to an external device, e.g., a user's smartphone, to display the result to a user. However, this is merely an example, and the communication interface may transmit and receive various necessary information.

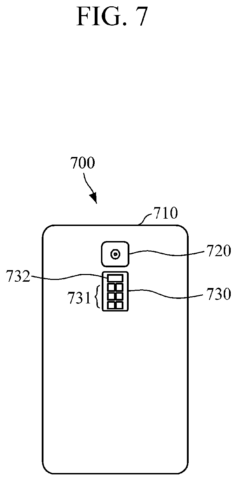

FIG. 7 is a diagram illustrating a smart device including a blood pressure estimating apparatus according to an example embodiment. Examples of the smart device may include a smartphone, a tablet PC, and the like.

Referring to FIG. 7, the smart device 700 includes a sensor part 730 mounted on one surface of a main body 710. The sensor part 730 may include a pulse wave sensor which includes one or more light sources 731 and a detector 732. As illustrated in FIG. 7, the sensor part 730 may be mounted on a rear surface of the main body 710, but is not limited thereto. Further, the sensor part 730 may be configured in combination with a fingerprint sensor or a touch panel mounted on a front surface.

In addition, a display may be mounted on a front surface of the main body 710. The display may visually display an estimation result of bio-information and the like. The display may include a touch panel, and may receive various types of information input through the touch panel and transmit the received information to the processor.

Moreover, an image sensor 720 may be mounted in the main body 710. The image sensor 720 may embodied as a camera and/or a fingerprint scanner. When a user's finger approaches the sensor part 730 to measure a pulse wave signal, the image sensor 720 may capture an image of the finger and may transmit the captured image to the processor. In particular, based on the image of the finger, the processor may identify a relative position of the finger with respect to an actual position of the sensor part 730, and may provide the relative position of the finger to the user through the display, so that pulse wave signals may be measured with improved accuracy.

The processor may independently estimate SBP and DBP by using the bio-signal measured by the sensor part 730. As described above in detail, the processor may obtain features, related to areas for estimating each of SBP and DBP, from the bio-signal, and may estimate SBP and DBP by using the obtained features related to the areas. Various modules for performing various other functions may be mounted in the smart device 700, and detailed description thereof will be omitted.

While not restricted thereto, an example embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

**Claims**

1. A blood pressure estimating apparatus (100a, 100b) comprising:

a sensor (100) configured to measure (410) a bio-signal waveform (30), said bio-signal corresponding to a photoplethysmogram, PPG, signal; and
a processor (120) configured to obtain (441) a first feature based on a first area under the bio-signal waveform in a first time interval, obtain (442) a second feature based on a second area under the bio-signal waveform in a second time interval which is different from the first time interval, and estimate a blood pressure based on the first feature and the second feature,
wherein the processor is further configured to determine a start time and an end time of each of the first time interval and the second time interval based on a bio-signal period of the bio-signal waveform and a reference starting value and a reference ending value of each of the first time interval and the second time interval, and adjust the start time and the end time of each of the first time interval and the second time interval in response to a rate of change in the bio-signal waveform over time being greater than a reference rate, and
wherein the processor is further configured to estimate a systolic blood pressure, SBP, by using the first feature and to estimate a diastolic blood pressure, DBP, by using the second feature.

2. The blood pressure estimating apparatus of claim 1, wherein the processor is further configured to estimate (451, 452) the systolic blood pressure, SBP, and the diastolic blood pressure, DBP, by applying a blood pressure estimation model to each of the first feature and the second feature.

3. The blood pressure estimating apparatus of claim 1or 2, wherein the reference starting value and the reference ending value of each of the first time interval and the second time interval comprise at least one of a constant value and a variable value which is adaptively changed according to a shape of the bio-signal waveform, or
wherein the processor (120) is further configured to obtain (431, 432) the first area of the first time interval and the second area of the second time interval by integrating differences between an amplitude value of the bio-signal waveform and an amplitude value of a baseline of the bio-signal waveform, for the first time interval and the second time interval, respectively.

4. The blood pressure estimating apparatus of claim 3, wherein the amplitude value of the baseline comprises at least one of an amplitude value of the bio-signal waveform at a measurement start time of the bio-signal waveform, a minimum amplitude value of the bio-signal waveform, and an amplitude value of a straight line formed by connecting the start time and the end time of each of the first time interval and the second time interval.

5. The blood pressure estimating apparatus of one of claims 1 to 4, wherein the processor is further configured to obtain the first feature and the second feature by normalizing the first area and the second area based on a first reference value and a second reference value, respectively.

**6.** The blood pressure estimating apparatus of one of claims 1 to 5, wherein the processor (120) is further configured to normalize the first area and the second area by dividing the first area and the second area by the first reference value and the second reference value, respectively, or by dividing the first reference value and the second reference value by the first area and the second area, respectively, or wherein the processor (120) is further configured to set the first reference value and the second reference value based on an amplitude value of the bio-signal waveform in a systolic phase or a diastolic phase of the bio-signal waveform, an amplitude value of an internally dividing point between two pulse waveform components of the bio-signal waveform, and a maximum amplitude value of the bio-signal waveform in the systolic phase or the diastolic phase.

**7.** The blood pressure estimating apparatus of one of claims 1 to 6, wherein the processor (120) is further configured to:

obtain a third feature (543) from the bio-signal waveform based on at least one of a shape of the bio-signal waveform, a time value and an amplitude value of a maximum point of the bio-signal waveform, a time value and an amplitude value of a minimum point of the bio-signal waveform, a time value and an amplitude value of a position of a pulse waveform component present in the bio-signal waveform, and an area under the bio-signal waveform in a third time interval;
estimate (551) a systolic blood pressure based on the first feature and the third feature; and
estimate (552) a diastolic blood pressure based on the second feature and the third feature.

**8.** A computer-implemented blood pressure estimating method comprising:

measuring (410) a bio-signal waveform (30), said bio-signal corresponding to a photoplethysmogram, PPG, signal;
obtaining (431, 432) a first area under the bio-signal waveform in a first time interval and a second area under the bio-signal waveform in a second time interval which is different from the first time interval, wherein a start time and an end time of each of the first time interval and the second time interval are determined based on a bio-signal period of the bio-signal waveform and a reference starting value and a reference ending value of each of the first time interval and the second time interval;
adjusting the start time and the end time of each of the first time interval and the second time interval in response to a rate of change in the bio-signal waveform over time being greater than a reference rate;
obtaining (441, 442) a first feature and a second feature based on the first area and the second area, respectively; and
estimating (451, 452) a blood pressure based on the first feature and the second feature, wherein the estimating a blood pressure comprises:
estimating a systolic blood pressure, SBP, by using the first feature and estimating a diastolic blood pressure, DBP, by using the second feature.

**9.** The blood pressure estimating method of claim 8,

wherein obtaining the first feature and the second feature comprises normalizing the first area and the second area based on a first reference value and a second reference value, respectively, or
further comprising obtaining (543) a third feature from the bio-signal waveform based on at least on one or two or more of a shape of the bio-signal waveform, a time value and an amplitude value of a maximum point of the bio-signal waveform, a time value and an amplitude value of a minimum point of the bio-signal waveform, a time value and an amplitude value of a position of a pulse waveform component present in the bio-signal waveform, and an area under the bio-signal waveform in a third time interval, wherein the estimating the blood pressure comprises estimating (551) a systolic blood pressure based on the first feature and the third feature, and estimating (552) a diastolic blood pressure based on the second feature and the third feature.

**10.** The blood pressure estimating method of claim 9, wherein the obtaining the first feature and the second feature comprises setting the first reference value and the second reference value based on at least one of an amplitude value of the bio-signal waveform in a systolic phase or a diastolic phase of the bio-signal waveform, an amplitude value of an internally dividing point between two pulse waveform components of the bio-signal waveform, and a maximum amplitude value of the bio-signal waveform in the systolic phase or the diastolic phase.

**11.** A non-transitory computer readable storage medium storing a program that is executable by a computer to perform the method of any of claims 8 to 10.

**Patentansprüche**

1. Blutdruckschätzvorrichtung (100a, 100b) mit:

   einem Sensor (100), der ausgebildet ist, eine Biosignalform (30) zu messen (410), wobei das Biosignal einem Photoplethysmogramm-, PPG-, Signal entspricht; und
   einem Prozessor (120), der ausgebildet ist, eine erste Eigenschaft basierend auf einer ersten Fläche unter der Biosignalform in einem ersten Zeitintervall zu erhalten (441), eine zweite Eigenschaft basierend auf einer zweiten Fläche unter der Biosignalform in einem zweiten Zeitintervall, das sich von dem ersten Zeitintervall unterscheidet, zu erhalten (442) und einen Blutdrucks basierend auf der ersten Eigenschaft und der zweiten Eigenschaft zu schätzen, wobei der Prozessor ferner ausgebildet ist, eine Startzeit und eine Endzeit des ersten Zeitintervalls und des zweiten Zeitintervalls basierend auf einer Biosignalperiode der Biosignalform und einem Referenzstartwert und einem Referenzendwert des ersten Zeitintervalls und des zweiten Zeitintervalls zu bestimmen und die Startzeit und die Endzeit jeweils des ersten Zeitintervalls und des zweiten Zeitintervalls in Reaktion auf eine Änderungsrate der Biosignalform im zeitlichen Verlauf, die größer als eine Referenzrate ist, einzustellen, und
   wobei der Prozessor ferner ausgebildet ist, einen systolischen Blutdruck, SBP, unter Verwendung der ersten Eigenschaft zu schätzen und einen diastolischen Blutdruck, DBP, unter Verwendung der zweiten Eigenschaft zu schätzen

2. Blutdruckschätzvorrichtung nach Anspruch 1, wobei der Prozessor ferner ausgebildet ist, den systolischen Blutdruck, SBP, und den diastolischen Blutdruck, DBP, zu schätzen (451, 452), indem ein Blutdruckschätzmodell jeweils auf die erste Eigenschaft und die zweite Eigenschaft angewendet wird.

3. Blutdruckschätzvorrichtung nach Anspruch 1 oder 2, wobei der Referenzstartwert und der Referenzendwert jeweils des ersten Zeitintervalls und des zweiten Zeitintervalls einen konstanten Wert und/oder einen variablen Wert umfassen, der entsprechend der Form der Biosignalform adaptiv geändert wird, oder
wobei der Prozessor (120) ferner ausgebildet ist, die erste Fläche des ersten Zeitintervalls und die zweite Fläche des zweiten Zeitintervalls durch Integrieren von Differenzen zwischen einem Amplitudenwert der Biosignalform und einem Amplitudenwert einer Grundlinie der Biosignalform entsprechend für das erste Zeitintervall und das zweite Zeitintervall zu ermitteln (431, 432).

4. Blutdruckschätzvorrichtung nach Anspruch 3, wobei der Amplitudenwert der Grundlinie umfasst: einen Amplitudenwert der Biosignalform zu einem Messungsstartzeitpunkt der Biosignalform und/oder einen minimalen Amplitudenwert der Biosignalform und/oder einen Amplitudenwert einer geraden Linie, die durch Verbinden des Startzeitpunkts und des Endzeitpunkts jeweils des ersten Zeitintervalls und des zweiten Zeitintervalls gebildet wird.

5. Blutdruckschätzvorrichtung nach einem der Ansprüche 1 bis 4, wobei der Prozessor weiterhin ausgebildet ist, die erste Eigenschaft und die zweite Eigenschaft durch Normalisierung der ersten Fläche und der zweiten Fläche auf der Grundlage entsprechend eines ersten Referenzwerts und eines zweiten Referenzwerts zu erhalten.

6. Blutdruckschätzvorrichtung nach einem der Ansprüche 1 bis 5, wobei der Prozessor (120) ferner ausgebildet ist, die erste Fläche und die zweite Fläche zu normalisieren, indem die erste Fläche und die zweite Fläche entsprechend durch den ersten Referenzwert und den zweiten Referenzwert geteilt werden, oder indem der erste Referenzwert und der zweite Referenzwert entsprechend durch die erste Fläche und die zweite Fläche geteilt werden, oder
wobei der Prozessor (120) ferner ausgebildet ist, den ersten Referenzwert und den zweiten Referenzwert auf der Grundlage eines Amplitudenwerts der Biosignalform in einer systolischen Phase oder einer diastolischen Phase der Biosignalform, eines Amplitudenwerts eines internen Teilungspunkts zwischen zwei Pulssignalkomponenten der Biosignalform, und eines maximalen Amplitudenwerts der Biosignalform in der systolischen Phase oder der diastolischen Phase festzulegen.

7. Blutdruckschätzvorrichtung nach einem der Ansprüche 1 bis 6, wobei der Prozessor (120) ferner ausgebildet ist zum:

   Erhalten einer dritten Eigenschaft (543) aus der Biosignalform auf der Grundlage einer Form der Biosignalform und/oder einem Zeitwert und einem Amplitudenwert eines Maximalpunkts der Biosignalform und/oder einem Zeitwert und einem Amplitudenwert eines Minimalpunkts der Biosignalform und/oder einem Zeitwert und einem Amplitudenwert einer Position einer Pulssignalkomponente, die in der Biosignalform vorhanden ist, und/oder einer Fläche unter der Biosignalform in einem dritten Zeitintervall;
   Schätzen (551) eines systolischen Blutdrucks auf der Grundlage der ersten Eigenschaft und der dritten Eigenschaft;
   und
   Schätzen (552) eines diastolischen Blutdrucks auf der Grundlage der zweiten Eigenschaft und der dritten Eigenschaft.

8. Computerimplementiertes Verfahren zur Blutdruckschätzung, das umfasst:

Messen (410) einer Biosignalform (30), wobei das Biosignal einem Photoplethysmogramm-, PPG-, Signal entspricht;

Erhalten (431, 432) einer ersten Fläche unter der Biosignalform in einem ersten Zeitintervall und einer zweiten Fläche unter der Biosignalform in einem zweiten Zeitintervall, das sich Von dem ersten Zeitintervall unterscheidet, wobei eine Startzeit und eine Endzeit jeweils des ersten Zeitintervalls und des zweiten Zeitintervalls auf der Grundlage einer Biosignalperiode der Biosignalform und eines Referenzstartwerts und eines Referenzendwerts jeweils des ersten Zeitintervalls und des zweiten Zeitintervalls bestimmt werden;

Einstellen der Startzeit und der Endzeit jeweils des ersten Zeitintervalls und des zweiten Zeitintervalls als Reaktion darauf, dass eine Änderungsrate der Biosignalform im zeitlichen Verlauf größer als eine Referenzrate ist;

Erhalten (441, 442) einer ersten Eigenschaft und einer zweiten Eigenschaft basierend auf entsprechend der ersten Fläche und der zweiten Fläche; und

Schätzen (451, 452) eines Blutdrucks auf der Grundlage der ersten Eigenschaft und der zweiten Eigenschaft, wobei das Schätzen eines Blutdrucks umfasst:

Schätzen eines systolischen Blutdrucks, SBP, unter Verwendung der ersten Eigenschaft und Schätzen eines diastolischen Blutdrucks, DBP, unter Verwendung der zweiten Eigenschaft.

9. Verfahren zur Blutdruckschätzung nach Anspruch 8,

wobei das Erhalten der ersten Eigenschaft und der zweiten Eigenschaft Normalisieren der ersten Fläche und der zweiten Fläche auf der Grundlage entsprechend eines ersten Referenzwerts und eines zweiten Referenzwerts umfasst oder

ferner umfasst: Erhalten (543) einer dritten Eigenschaft aus der Biosignalform auf der Grundlage von mindestens einem oder zwei oder mehr der folgenden Aspekte: einer Form der Biosignalform, einem Zeitwert und einem Amplitudenwert eines Maximalpunkts der Biosignalform, eines Zeitwerts und eines Amplitudenwerts eines Minimalpunkts der Biosignalform, eines Zeitwerts und eines Amplitudenwerts einer Position einer in der Biosignalform vorhandenen Pulssignalkomponente und einer Fläche unter der Biosignalform in einem dritten Zeitintervall, wobei das Schätzen des Blutdrucks Schätzen (551) eines systolischen Blutdrucks auf der Grundlage der ersten Eigenschaft und der dritten Eigenschaft und Schätzen (552) eines diastolischen Blutdrucks auf der Grundlage der zweiten Eigenschaft und der dritten Eigenschaft umfasst.

10. Verfahren zur Blutdruckschätzung nach Anspruch 9, wobei das Erhalten der ersten Eigenschaft und der zweiten Eigenschaft Festlegen des ersten Referenzwerts und des zweiten Referenzwerts auf der Grundlage eines Amplitudenwerts der Biosignalform in einer systolischen Phase oder einer diastolischen Phase der Biosignalform, und/oder eines Amplitudenwerts eines internen Teilungspunkts zwischen zwei Pulssignalkomponenten der Biosignalform, und/oder eines maximalen Amplitudenwerts der Biosignalform in der systolischen Phase oder der diastolischen Phase umfasst.

11. Nicht-flüchtiges, computerlesbares Speichermedium, das ein Programm speichert, das von einem Computer ausführbar ist, um das Verfahren gemäß einem der Ansprüche 8 bis 10 durchzuführen.

**Revendications**

1. Appareil d'estimation de la tension artérielle (100a, 100b) comprenant :

un capteur (100) configuré pour mesurer (410) une forme d'onde de bio-signal (30), ledit bio-signal correspondant à un signal de photopléthysmographie, PPG ; et

un processeur (120) configuré pour obtenir (441) une première caractéristique sur la base d'une première zone sous la forme d'onde de bio-signal dans un premier intervalle de temps, obtenir (442) une seconde caractéristique sur la base d'une seconde zone sous la forme d'onde de bio-signal dans un second intervalle de temps qui est différent du premier intervalle de temps, et pour estimer une tension artérielle sur la base de la première caractéristique et de la seconde caractéristique,

dans lequel le processeur est en outre configuré pour déterminer un temps de début et un temps de fin de chacun du premier intervalle de temps et du second intervalle de temps sur la base d'une période de bio-signal de la forme d'onde de bio-signal et d'une valeur de début de référence et d'une valeur de fin de référence de chacun du premier intervalle de temps et du second intervalle de temps, et ajuster le temps de début et le temps de fin de chacun du premier intervalle de temps et du second intervalle de temps en réponse à un taux de changement dans la forme d'onde de bio-signal dans le temps qui est supérieur à un taux de référence, et

dans lequel le processeur est en outre configuré pour estimer une tension artérielle systolique, SBP, en utilisant la première caractéristique et pour estimer une tension artérielle diastolique, DBP, en utilisant la seconde caractéristique.

2. Appareil d'estimation de la tension artérielle selon la revendication 1, dans lequel le processeur est en outre configuré pour estimer (451, 452) la tension artérielle systolique, SBP, et la tension artérielle diastolique, DBP, en appliquant un modèle d'estimation de la tension artérielle à chacune de la première caractéristique et de la seconde caractéristique.

3. Appareil d'estimation de la tension artérielle selon la revendication 1 ou 2, dans lequel la valeur de début de référence et la valeur de fin de référence de chacun du premier intervalle de temps et du second intervalle de temps comprennent au moins l'une d'une valeur constante et d'une valeur variable qui est modifiée de manière adaptative en fonction d'une forme de la forme d'onde de bio-signal, ou dans lequel le processeur (120) est en outre configuré pour obtenir (431, 432) la première zone du premier intervalle de temps et la seconde zone du second intervalle de temps en intégrant des différences entre une valeur d'amplitude de la forme d'onde de bio-signal et une valeur d'amplitude d'une ligne de référence de la forme d'onde de bio-signal, pour le premier intervalle de temps et le second intervalle de temps, respectivement.

4. Appareil d'estimation de la tension artérielle selon la revendication 3, dans lequel la valeur d'amplitude de la ligne de référence comprend au moins l'une d'une valeur d'amplitude de la forme d'onde de bio-signal à un temps de début de mesure de la forme d'onde de bio-signal, une valeur d'amplitude minimale de la forme d'onde de bio-signal, et une valeur d'amplitude d'une ligne droite formée en raccordant le temps de début et le temps de fin de chacun du premier intervalle de temps et du second intervalle de temps.

5. Appareil d'estimation de la tension artérielle selon l'une des revendications 1 à 4, dans lequel le processeur est en outre configuré pour obtenir la première caractéristique et la seconde caractéristique en normalisant la première zone et la seconde zone sur la base d'une première valeur de référence et d'une seconde valeur de référence, respectivement.

6. Appareil d'estimation de la tension artérielle selon l'une des revendications 1 à 5, dans lequel le processeur (120) est en outre configuré pour normaliser la première zone et la seconde zone en divisant la première zone et la seconde zone par la première valeur de référence et la seconde valeur de référence, respectivement, ou en divisant la première valeur de référence et la seconde valeur de référence par la première zone et la seconde zone, respectivement, ou dans lequel le processeur (120) est en outre configuré pour définir la première valeur de référence et la seconde valeur de référence sur la base d'une valeur d'amplitude de la forme d'onde de bio-signal dans une phase systolique ou une phase diastolique de la forme d'onde de bio-signal, d'une valeur d'amplitude d'un point de division au plan interne entre deux composantes de la forme d'onde d'impulsion de la forme d'onde de bio-signal, et d'une valeur d'amplitude maximale de la forme d'onde de bio-signal dans la phase systolique ou la phase diastolique.

7. Appareil d'estimation de la tension artérielle selon l'une des revendications 1 à 6, dans lequel le processeur (120) est en outre configuré pour :

obtenir une troisième caractéristique (543) à partir de la forme d'onde de bio-signal sur la base d'au moins l'une d'une forme de la forme d'onde de bio-signal, d'une valeur de temps et d'une valeur d'amplitude d'un point maximal de la forme d'onde de bio-signal, d'une valeur de temps et d'une valeur d'amplitude d'un point minimal de la forme d'onde de bio-signal, d'une valeur de temps et d'une valeur d'amplitude d'une position d'une composante de forme d'onde d'impulsion présente dans la forme d'onde de bio-signal, et d'une zone sous la forme d'onde de bio-signal dans un troisième intervalle de temps ;
estimer (551) une tension artérielle systolique sur la base de la première caractéristique et de la troisième caractéristique ; et
pour estimer (552) une tension artérielle diastolique sur la base de la seconde caractéristique et de la troisième caractéristique.

8. Procédé mis en œuvre par ordinateur d'estimation de la tension artérielle comprenant les étapes consistant à :

mesurer (410) une forme d'onde de bio-signal (30), ledit bio-signal correspondant à un signal de photopléthysmographie, PPG ;
obtenir (431, 432) une première zone sous la forme d'onde de bio-signal dans un premier intervalle de temps et une seconde zone sous la forme d'onde de bio-signal dans un second intervalle de temps qui est différent du premier intervalle de temps, dans lequel un temps de début et un temps de fin de chacun du premier intervalle de temps et du second intervalle de temps sont déterminés sur la base d'une période de bio-signal de la forme d'onde de bio-signal et d'une valeur de début de référence et d'une valeur de fin de référence de chacun du premier intervalle de temps et du second intervalle de temps ;

ajuster le temps de début et le temps de fin de chacun du premier intervalle de temps et du second intervalle de temps en réponse à un taux de changement dans la forme d'onde de bio-signal dans le temps qui est supérieur à un taux de référence ;

obtenir (441, 442) une première caractéristique et une seconde caractéristique sur la base de la première zone et de la seconde zone, respectivement ; et

à estimer (451, 452) une tension artérielle sur la base de la première caractéristique et de la seconde caractéristique,

dans lequel l'estimation d'une tension artérielle comprend l'étape consistant à :

estimer une tension artérielle systolique, SBP, en utilisant la première caractéristique et à estimer une tension artérielle diastolique, DBP, en utilisant la seconde caractéristique.

9. Procédé d'estimation de la tension artérielle selon la revendication 8,

dans lequel l'obtention de la première caractéristique et de la seconde caractéristique comprend la normalisation de la première zone et de la seconde zone sur la base d'une première valeur de référence et d'une seconde valeur de référence, respectivement, ou comprenant en outre l'étape consistant à obtenir une troisième caractéristique (543) à partir de la forme d'onde de bio-signal sur la base d'au moins l'une ou deux ou plus d'une forme de la forme d'onde de bio-signal, d'une valeur de temps et d'une valeur d'amplitude d'un point maximal de la forme d'onde de bio-signal, d'une valeur de temps et d'une valeur d'amplitude d'un point minimal de la forme d'onde de bio-signal, d'une valeur de temps et d'une valeur d'amplitude d'une position d'une composante de forme d'onde d'impulsion présente dans la forme d'onde de bio-signal, et d'une zone sous la forme d'onde de bio-signal dans un troisième intervalle de temps, dans lequel l'estimation de la tension artérielle comprend l'étape d'estimation (551) d'une tension artérielle systolique sur la base de la première caractéristique et de la troisième caractéristique, et l'étape d'estimation (552) d'une tension artérielle diastolique sur la base de la seconde caractéristique et de la troisième caractéristique.

10. Procédé d'estimation de la tension artérielle selon la revendication 9, dans lequel l'obtention de la première caractéristique et de la seconde caractéristique comprend la définition de la première valeur de référence et de la seconde valeur de référence sur la base d'au moins l'une d'une valeur d'amplitude de la forme d'onde de bio-signal dans une phase systolique ou une phase diastolique de la forme d'onde de bio-signal, d'une valeur d'amplitude d'un point de division au plan interne entre deux composantes de la forme d'onde d'impulsion de la forme d'onde de bio-signal, et d'une valeur d'amplitude maximale de la forme d'onde de bio-signal dans la phase systolique ou la phase diastolique.

11. Support de stockage non temporaire, lisible par ordinateur stockant un programme qui peut être exécuté par un ordinateur pour exécuter le procédé selon l'une quelconque des revendications 8 à 10.

FIG. 1A

100a

110

SENSOR

120

PROCESSOR

FIG. 1B

```
                        ┌─ 100b
  ┌ ─ ─ ─ ─ ─ ─ ─ ─ ─ ─ ─ ─ ─ ─ ─ ─ ─ ─ ─ ─ ─ ┐
  |          ┌─ 110                            |
  |  ┌─────────────────────┐                   |
  |  │      SENSOR         │                   |
  |  └─────────────────────┘                   |
  |           ↕                                |
  |          ┌─ 120                            |
  |  ┌─────────────────────┐   ┌──────────┐    |   ┌──────────┐
  |  │     PROCESSOR       │↔ │COMMUNI-  │    ↔  │EXTERNAL  │
  |  └─────────────────────┘   │CATION    │    |   │DEVICE    │
  |        ↕         ↕          │INTERFACE │    |   └──────────┘
  |   ┌─130     ┌─140          └──────────┘    |      ┌─ 170
  | ┌────────┐ ┌────────┐       ┌─ 150         |
  | │OUTPUT  │ │STORAGE │                      |
  | │INTERFACE│ │        │                     |
  | └────────┘ └────────┘                      |
  └ ─ ─ ─ ─ ─ ─ ─ ─ ─ ─ ─ ─ ─ ─ ─ ─ ─ ─ ─ ─ ─ ┘
```

# FIG. 2A

200a

210

211

FIRST AREA
OBTAINER

212

SECOND AREA
OBTAINER

BIO-
SIGNAL

220

221

FIRST
FEATURE
OBTAINER

222

SECOND
FEATURE
OBTAINER

230

231

SBP
ESTIMATOR

232

DBP
ESTIMATOR

# FIG. 2B

200b

# FIG. 3A

# FIG. 3B

# FIG. 3C

AMPLITUDE

PPG SIGNAL

Adia

TIME

$\tau\_dia\_1xT\_period$     $\tau\_dia\_2xT\_period$

FIG. 3D

# FIG. 3E

# FIG. 4

START

RECEIVE REQUEST FOR
ESTIMATING BLOOD PRESSURE ⟋410

MEASURE BIO-SIGNAL ⟋420

OBTAIN AREA OF FIRST
INTERVAL OF BIO-SIGNAL ⟋431

OBTAIN AREA OF SECOND
INTERVAL OF BIO-SIGNAL ⟋432

OBTAIN FIRST FEATURE BASED
ON AREA OF FIRST INTERVAL ⟋441

OBTAIN SECOND FEATURE BASED
ON AREA OF SECOND INTERVAL ⟋442

ESTIMATE SBP BASED ON
FIRST FEATURE ⟋451

ESTIMATE DBP BASED ON
SECOND FEATURE ⟋452

OUTPUT ESTIMATION RESULT
OF BLOOD PRESSURE ⟋460

END

# FIG. 5

```
                    ┌─────────┐
                    │  START  │
                    └────┬────┘
                         │        ╭510
            ┌────────────▼────────────┐
            │   RECEIVE REQUEST FOR   │
            │ ESTIMATING BLOOD PRESSURE│
            └────────────┬────────────┘
                         │        ╭520
            ┌────────────▼────────────┐
            │    MEASURE BIO-SIGNAL    │
            └────────────┬────────────┘
```

OBTAIN AREA OF FIRST INTERVAL OF BIO-SIGNAL — 531

OBTAIN AREA OF SECOND INTERVAL OF BIO-SIGNAL — 532

OBTAIN FIRST FEATURE BASED ON AREA OF FIRST INTERVAL — 541

OBTAIN THIRD FEATURE BASED ON BIO-SIGNAL — 543

OBTAIN SECOND FEATURE BASED ON AREA OF SECOND INTERVAL — 542

ESTIMATE SBP BASED ON FIRST FEATURE AND THIRD FEATURE — 551

ESTIMATE DBP BASED ON SECOND FEATURE AND THIRD FEATURE — 552

OUTPUT ESTIMATION RESULT OF BLOOD PRESSURE — 560

END

EP 3 636 145 B1

# FIG. 6A

# FIG. 6B

614

♥ BLOOD PRESSURE INFORMATION ♥
118/78

MEASUREMENT DATE: 2018-05-15 10:00
HEALTH CONDITION: GOOD

614a

614b

M    I

# FIG. 7

**EP 3 636 145 B1**

**Patent documents cited in the description**

- US 2011288422 A1
- US 2005119578 A1
- CN 107233087 A
- US 2005261593 A1